# EUROPEAN PATENT APPLICATION

(11) **EP 2 354 159 A1**
(43) Date of publication of application: **10.08.2011**
(21) Application number: 10001208.7
(22) Date of filing: 05.02.2010
(51) Int. Cl.: C07K 16/24, C07K 14/52, C07K 14/775

(54) **CCL17 inhibitors for use in T helper cell-driven diseases**

(71) Applicant: RWTH Aachen, 52062 Aachen (DE)
(72) Inventor: Zernecke, Alma, Dr., 52074 Aachen (DE); Weber, Christian, Dr., 52074 Aachen (DE)
(74) Representative: Michalski Hüttermann & Partner Patentanwälte

(57) **Abstract**

The present invention is based on the studies of the pathogenic role of CCL17-expressing DCs and their effector function in inflammatory diseases, particularly in atherosclerotic diseases. It relates to agents inhibiting the binding reaction between a CCL17 cytokine and a CCL17 receptor for use as a medicament (Fig. 5a).

## Description

### Field of the invention

The present invention relates to inhibitors of the cytokine CCL 17 and the corresponding receptors and its use as therapeutic in T helper cell-driven diseases.

### Background of the invention .

Chronic inflammatory diseases often cause specific immune responses. Atherosclerosis is such a chronic inflammatory disease in which an artery wall thickens as the result of a build-up of fatty materials such as cholesterol. It is a syndrome affecting arterial blood vessels, a chronic inflammatory response in the walls of arteries, in large part due to the accumulation of macrophage white blood cells and promoted by Low-density lipoproteins (plasma proteins that carry cholesterol and triglycerides) without adequate removal of fats and cholesterol from the macrophages by functional high density lipoproteins (HDL).

Other inflammatory diseases which are related to the immune system are autoimmune diseases. These diseases arise from an overactive immune response against substances and tissues normally present in the body. As a result the body attacks its own cells and mistakes parts of the body as pathogens. This may be restricted to certain organs (e.g. in thyroiditis) or involve a particular tissue in different places (e.g. Goodpasture's disease which may affect the basement membrane in both the lung and the kidney). Typically, autoimmune diseases are treated with immunosuppressive medication. In both autoimmune and inflammatory diseases the condition arises through aberrant reactions of the human adaptive or innate immune systems. In autoimmunity, the patient's immune system is activated against the body's own proteins. In inflammatory diseases, it is the overreaction of the immune system, and its subsequent downstream signaling (TNF, IFN, etc), which causes problems.

When referring to atherosclerotic lesions there are besides monocytes/macrophages, also T helper cells (particularly Th-1-cells) and Dendritic cells (DCs) detectable. DCs are professignal antigen-presenting cells that can be divided into several heterogeneous subtypes and are essential for priming of immune responses. A network of DCs has been identified in the arterial intima of healthy young individuals and an accumulation of DCs can be observed in the intima and adventitia of atherosclerosis-susceptible regions in mice. In advanced human plaques increased numbers of DCs are found within rupture-prone regions in clusters with T cells. The DC-derived chemokine CCL17, also known as TARC*lthymus- and activation-regulated chemokine,* is expressed in such atherosclerotic lesions.

Modified lipoproteins deposited in the arterial wall may constitute antigens that instigate an early immune activation of vascular DCs. Consequently, antigen-specific and clonally expanded T cells reactive for modified lipoproteins were found in early plaques of patients. These findings imply DCs and their products, such as CCL17, to play in important rule in the pathogenesis of inflammatory diseases such as atherosclerosis.

Other causes for artherosclerotic diseases comprise a respective genetic predisposition, physical condition of the patient, smoking, degree of physical exercise the patient is currently undergoing, or infectious diseases caused, e.g., by herpes virus or cytomegaly virus.

T helper cells (particularly Th-1-cells) play also a significant role in autoimmune diseases.

They are a sub-group of lymphocytes that play an important role in establishing and maximizing the capabilities of the immune system. T helper cells are unusual in that they have no cytotoxic or phagocytic activity, but they are involved in activating and directing other immune cells. They are essential in determining B cell antibody class switching, in the activation and growth of cytotoxic T cells, and in maximizing bactericidal activity of phagocytes such as macrophages. Considering the diverse and important role helper T cells play in the immune system, it is not surprising that these cells achieve a balance of sensitivity in order to respond to foreign antigens without responding to the antigens of the host itself. When the immune system responds to very low levels of antigen that it usually shouldn't respond to, a hypersensitivity response occurs which is believed to be the cause of allergy and some auto-immune diseases. Dardalhon et al (2008) have reported that Th1 cells have been associated with the pathogenesis of autoimmune diseases, although other effector T cells, like Th17 cells, are also considered to be responsible for inducing autoimmunity.

### Object of the invention

The present invention is based on the studies of the pathogenic role of CCL17-expressing DCs and their effector function in inflammatory diseases, particularly in artheroslecoris and autoimmune diseases. The object of the present invention predicted on these studies is to provide a medicament for the therapy of such diseases.

This object is met with means according to the independent claims of the present invention. The dependent claims are related to preferred embodiments.

### Summary of the invention

Before the invention is described in detail, it is to be understood that this invention is not limited to the particular biological systems. The described biological systems may vary. It is also to be understood that the terminology used herein is for purposes of describing particular embodiments only, and is not intended to be limiting. It must be noted that, as used in the specification and the appended claims, the singular forms "a", "an" and "the" include singular and/or plural referents unless the context clearly dictates otherwise. It is moreover to be understood that, in case parameter ranges are given which are delimited by numeric values, the ranges are deemed to include these limitation values.

In the first aspect, the present invention relates to an agent inhibiting the binding reaction between a CCL17 cytokine and a CCL17 receptor for use as a medicament.

The inventors of the present invention have for the first described the role of CCL17 in the genesis of T helper cell Th1-driven diseases, particular artherosclerosis and autoimmune diseases. This finding came quite surprisingly.

Functionally, cytokines like CCL17 can be divided into two kinds; inflammatory and homeostatic (i.e, regulatory). Inflammatory cytokine s are induced to high levels of expression by inflammatory stimuli and play important roles in the development of immune/ /inflammatory responses by attracting inflammatory cells. In contrast, homeostatic cytokines are usually constitutively expressed by certain cell types in a restricted tissues and critically involved in the homeostatic regulation of lymphocyte trafficking and functional compartmentalization of lymphoid organs.

So far, CCL17 has been considered as a homeostatic cytokine, because it was known that it attracts inflammatory cells inducing cellular immune responses, like Th1 and Th17, as well as regulatory T cells (Treg) and T-cells inducing humoral immunity, like Th2. Furthermore, it was assumed so far that the major receptor for CCL17, CCR4, is mainly located on Th2 cells. Perros et al. (2009) have for example shown that human Th2 cells express the CCR4 chemokine receptor and that DC-derived CCL17 together with CCL22 plays a critical role in attracting Th2-polarized cells.

The findings of the inventors suggest that, in contrast to what has been though before, the cytokine CCL17 limits antigen-specific T-cell proliferation, promotes the polarization of T helper type 1 Th1/Th17 over Th2 cells and suppresses the expansion and maintenance of regulatory T (Treg) cells. These findings furthermore suggest that CCL17 may act, under certain circumstances, as inflammatory cytokine.

Preferably, said medicament is thus a medicament for treating a T helper cell Th1-driven disease. More preferably, the medicament is a medicament for treating an atherosclerotic disease with at least one pathologic condition selected from the group consisting of
● atherosclerosis
● myocardial infarction
● stroke
● peripheral vascular disease
● chronic kidney disease, and/or
● angina pectoris.

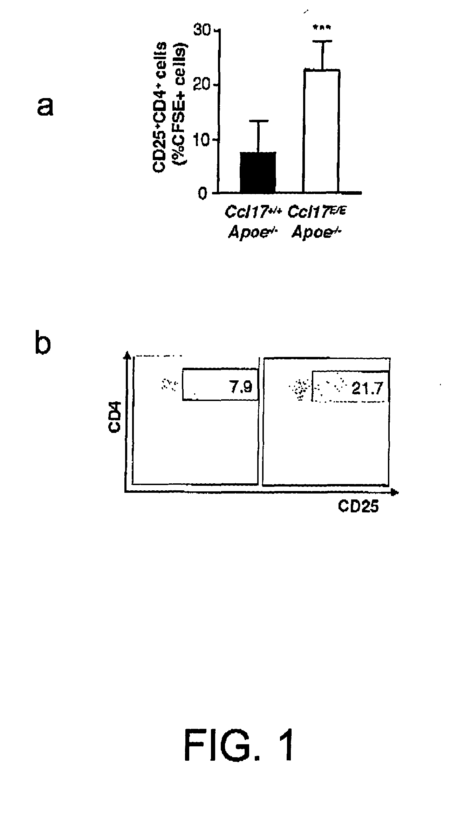

Data which support this indication are given, among others, in Examples I to VII and Fig. 1 to 17.

In another preferred embodiment, the medicament is a medicament for treating an autoimmune disease with at least one pathologic condition selected from the group consisting of
● hypersensitivity dermatitis (CHS)
● psoriasis
● rheumatoid arthritis;
● morbus bechterew and/or
● inflammatory bowel disease (morbus krohn)

Data which support this indication are given, among others, in Example VIII and Fig, 18

In another preferred embodiment, the CCL17 receptor is at least one receptor selected from the group consisting of CCR4 and/or CCR8.

Preferably, the agent is at least one selected from the group consisting of
● a CCL17 inhibitor,
● an antagonist and/or inhibitor against a CCL17 receptor, preferably against CCR4 and/or CCR8,
● a protein expression inhibitor capable of inhibiting the expression of at least one gene encoding for CCL17 and/or a CCL17 receptor, preferably CCR4 and/or CCR8, and/or
● a degrading agent capable of degrading CCL17 and/or a CCL17 receptor, preferably CCR4 and/or CCR8.

More preferably, the agent is a monoclonal antibody (mAb) and most preferably the monoclonal antibody is at least one selected from the group consiting of
● murine, chimeric, humanized and/or human mAb,
● IgG, F(ab)2, Fab, and/or scFv, and/or
● modified antibody format.

In other preferred embodiments the agent is at least one non-antibody-based target binding entity, a pan receptor antagonist or a small molecular drug.

In another aspect of the invention the agent is used for the preparation of a medicament.

In yet another aspect, the present invention relates to a non-human mammal being partially or completely deficient in expressing the non-human genes encoding CCL17 and apolipoprotein E. In a preferred embodiment of this aspect the deficiency is due to genetic alteration based on a homozygous or heterozygous mutation in the non-human genes encoding CCL17 and apolipoprotein E; or a homozygous or heterozygous deletion of the non-human genes encoding CCL17 and apolipoprotein E.

### Detailed description of the invention:

The present invention relates to agents inhibiting the reaction between a CCL17 cytokine and a CCL17 receptor for use as a medicament.

Cytokines are a number of substances that are secreted by specific cells of the immune system which carry signals locally between cells, and thus have an effect on other cells. They are a category of signaling molecules that are used extensively in cellular communication. They are proteins, peptides, or glycoproteins. The term cytokine encompasses a large and diverse family of polypeptide regulators that are produced widely throughout the body by cells of diverse embryological origin.

Chemokines are a family of small cytokines, or proteins secreted by cells. Their name is derived from their ability to induce directed chemotaxis in nearby responsive cells; they are chemotactic cytokines. Proteins are classified as chemokines according to shared structural characteristics such as small size (they are all approximately 8-10 kilodaltons in size), and the presence of four cysteine residues in conserved locations that are key to forming their 3-dimensional shape. However, these proteins have historically been known under several other names including the SIS family of cytokines, SIG family of cytokines, SCY family of cytokines, Platelet factor-4 superfamily or intercrines. Some chemokines are considered proinflammatory and can be induced during an immune response to promote cells of the immune system to a site of infection, while others are considered homeostatic and are involved in controlling the migration of cells during normal processes of tissue maintenance or development. Chemokines are found in all vertebrates, some viruses and some bacteria, but none have been described for other invertebrates. These proteins exert their biological effects by interacting with G protein-linked transmembrane receptors called chemokine receptors, that are selectively found on the surfaces of their target cells.

CCL17 ("Chemokine (C-C motif) ligand 17") is a small cytokine belonging to the CC chemokine family that is also known as thymus and activation regulated chemokine ("TARC"). CCL17 is expressed constitutively in thymus, but only transiently in phytohemagglutinin-stimulated peripheral blood mononuclear cells. This chemokine specifically binds and induces chemotaxis in T cells and elicits its effects by interacting with the chemokine receptors CCR4 and CCR8. The gene for CCL17 is located on chromosome 16, in humans, along with other chemokines called CCL22 and CX3CL1.

Despite the fact that CCL17 is already well described, its exact role has so far been unclear. In particular, an involvement in pathologic conditions has not been described yet.

The present invention shows for the first time a causal relationship between the presence of CCL17 in the blood flow and the occurrence of pathologic conditions as, but not limited to, atherosclerotic diseases, like atherosclerosis, myocardial infarction, stroke, peripheral vascular disease, chronic kidney disease, and/or angina pectoris or autoimmune disease, like hypersensitivity dermatitis (CHS), psoriasis, rheumatoid arthritis, morbus bechterew and/or inflammatory bowel disease (morbus krohn).

In the respect of artherosclerotic diseases, it is demonstrated that Dendritic cells ("DCs") expressing CCL17 accumulate in Arteriosclerotic lesions. Dendritic cells (DCs) are immune cells that form part of the mammalian immune system. Their main function is to process antigen material and present it on the surface to other cells of the immune system, thus functioning as antigen-presenting cells and as such essential for the priming of an immune response.

The pathogenic role of CCL17-epxressing DCs and their effector function in atherosclerosis is studied in knock-in mouse expressing a targeted replacement of the CCL17 gene by enhanced green fluorescent protein (Egfp, CCL17E/E). Based on the findings that CCL17 expression is up-regulated in human atherosclerotic arteries, a bone marrow (BM)-derived mature CCL17+ DC-subpopulation is identified, which controls T-cell homeostasis by suppressing Regulatory T (Treg)-cell proliferation and enhancing proinflammatory Th1- and Thl7-like T-cell responses, and which accumulates in atherosclerotic lesions during disease progression.

For knock-out experiments a apolipoprotein E-deficient mouse model *(Apoe* -/-) is used. This model quickly develops symptoms of atherosclerosis due to a deficiency of apolipoprotein E. Experimental data show that if the CCL17-gene is knocked out in this model, too *(Apoe -*/*-* /*CCL17-1-* ), atherosclerosis symptoms are significantly reduced. Genetic deletion of CCL17 (or its T-cell receptor) in *Apoe-l-* mice or antibody-blockade of CCL17 reduces development and progression of atherosclerosis in various models. The data clearly establish that DCs and their proinflammatory effector functions promote artherogenesis and that DC-derived CCL17 serves as a feasible therapeutic target.

With respect to the mouse model, the present invention also relates to non-human mammal being partially or completely deficient in expressing the non-human genes encoding CCL17 and apolipoprotein E. The deficiency is due to genetic alteration based on a homozygous or heterozygous mutation in the non-human genes encoding CCL17 and apolipoprtein E; or a homozygous or heterozygeous deletion of the non-human genes encoding CCL17 and apoli-poprotein E.

According to common scientific sense, the evidence shown here is already a clear indication that a therapy interfering in the CCL17 signalling pathway, e.g. by adminstration of an agent inhibiting the binding reaction between a CCL17 cytokine and a CCL17 receptor, is a promising therapy against the development of inflammatory diseases, such as atherosclerosis. As so far a use of an anti-CCL17 agent as therapeutic treatment has not been described, the suggested agent is a clear first medical indication.

The agent used as medicament is a medicament for treating an atherosclerotic disease with at least at least one pathologic condition selected from the group consisting of atherosclerosis, myocardial infarction, stroke, peripheral vascular disease, chronic kidney disease, and/or angina pectoris.

Moreover, the agent used as medicament is a medicament for treating an autoimmune disease with at least one pathologic condition selected from the group consisting of hypersensitivity dermatitis (CHS), psoriasis, rheumatoid arthritis, morbus bechterew, and/or inflammatory bowel disease (morbus krohn).

Atherosclerosis (also known as Arteriosclerotic Vascular Disease or ASVD) is a condition in which an artery wall thickens as the result of a build-up of fatty materials such as cholesterol. It is a syndrome affecting arterial blood vessels, a chronic inflammatory response in the walls of arteries, in large part due to the accumulation of macrophage white blood cells and promoted by Low-density lipoproteins (plasma proteins that carry cholesterol and triglycerides) without adequate removal of fats and cholesterol from the macrophages by functional high density lipoproteins (HDL). It is commonly referred to as a hardening or furring of the arteries and caused by the formation of multiple plaques within the arteries.

There are two known CCL17 receptors: CCR4 and CCR8. CCR4 belongs to the G protein-coupled receptor family of seven transmembrane domain receptors. CCR8 is a member of the beta chemokine receptor family, which is also a seven transmembrane protein similar to G protein-coupled receptors.

These chemokine receptors are G protein-coupled receptors containing 7 transmembrane domains that are found predominantly on the surface of leukocytes. They are composed of about 350 amino acids that are divided into a short and acidic N-terminal end, seven helical transmembrane domains with three intracellular and three extracellular hydrophilic loops, and an intracellular C-terminus containing serine and threonine residues that act as phosphorylation sites during receptor regulation. The first two extracellular loops of chemokine receptors are linked together by disulfide bonding between two conserved cysteine residues. The N-terminal end of a chemokine receptor binds to chemokine(s) and is important for ligand specificity. G-proteins couple to the C-terminal end, which is important for receptor signaling following ligand binding. Although chemokine receptors share high amino acid identity in their primary sequences, they typically bind a limited number of ligands.

Intracellular signaling by chemokine receptors is dependent on neighbouring G-proteins. G-proteins exist as a heterotrimer; they are composed of three distinct subunits. When the molecule GDP is bound to the G-protein subunit, the G-protein is in an inactive state. Following binding of the chemokine ligand, chemokine receptors associate with G-proteins, allowing the exchange of GDP for another molecule called GTP, and the dissociation of the different G protein subunits. The subunit called Gβ activates an enzyme known as Phospholipase C (PLC) that is associated with the cell membrane. PLC cleaves Phosphatidylinositol (4,5)-bisphosphate (PIP2) to form two second messenger molecules called inositol triphosphate (IP3) and diacylglycerol (DAG); DAG activates another enzyme called protein kinase C (PKC), and IP3 triggers the release of calcium from intracellular stores. These events promote many signaling cascades, effecting a cellular response. For example, when some cytokines bind to their specific receptors, a rise in intracellular calcium activates the enzyme phospholipase D (PLD) that goes on to initiate an intracellular signaling cascade called the MAP kinase pathway. At the same time the G-protein subunit Gα directly activates an enzyme called protein tyrosine kinase (PTK), which phosphorylates serine and threonine residues in the tail of the chemokine receptor causing its desensitisation or inactivation. The initiated MAP kinase pathway activates specific cellular mechanisms involved in chemotaxis, degranulation, release of superoxide anions and changes in the avidity of cell adhesion molecules called integrins.

According to the named receptors of CCL17, the agent inhibiting binding reaction between the CCL17 cytokine and its receptors CCR4 and/or CCR8 is at least one selected from the group consisting of
● a CCL17 inhibitor,
● an antagonist and/or inhibitor against a CCL17 receptor, preferably against CCR4 and/or CCR8,
● a protein expression inhibitor capable of inhibiting the expression of at least one gene encoding for CCL17 and/or a CCL17 receptor, preferably CCR4 and/or CCR8, and/or
● a degrading agent capable of degrading CCL17 and/or a CCL17 receptor, preferably CCR4 and/or CCR8.

As used herein, the term "CCL17 inhibitor" refers to entities which are able of binding to CCL17 and thus avoid binding of the latter by a CCL17 receptor. The said inhibitor does thus - inhibit the binding reaction between a CCL17 cytokine and a CCL17 receptor, and consequently avoids the production of a ligand-triggered response in the CCL17 receptor.

As used herein, the term "receptor inhibitor" refers to entities which are able of binding to a CCL17 receptor and thus avoid binding of CCL17 to the latter. The said inhibitor does thus inhibit the binding reaction between a CCL17 cytokine and a CCL17 receptor, and consequently avoids the production of a ligand-triggered response in the CCL17 receptor.

As used herein, the term "receptor antagonist" shall refer to a type of receptor ligand or drug that does not provoke a biological response itself upon binding to a receptor, but blocks or dampens agonist-mediated responses. Antagonists have affinity but no efficacy for their receptors, and binding will disrupt the interaction and inhibit the function of an agonist or inverse agonist at receptors. Antagonists mediate their effects by binding to the active site or to allosteric sites on receptors, or they may interact at unique binding sites not normally involved in the biological regulation of the receptor's activity. Antagonist activity may be reversible or irreversible depending on the longevity of the antagonist—receptor complex, which, in turn, depends on the nature of antagonist receptor binding. The majority of drug antagonists achieve their potency by competing with endogenous ligands or substrates at structurally-defined binding sites on receptors. The said antagonist does thus inhibit the binding reaction between a CCL17 cytokine and a CCL17 receptor, and consequently avoids the production of a ligand-triggered response in the CCL17 receptor.

As used herein, the term "protein expression inhibitor" refers to entities which inhibit the expression of at least one gene encoding for CCL17 and/or a CCL17 receptor, preferably CCR4 and/or CCR8. Such expression inhibitor is for example an antisense DNA and/or antisense mRNA construct.

As used herein, the term "degrading agent" refers to entities which decompose specific bindings within a target substrate related to the targeted disease. Such a degrading agent is for example a target specific protease with a defined specificity that is capable to hydrolyze specific peptide bonds within the CCL 17 cytokine or its receptors CCR4 or CCR8.

In a specific embodiment the invention provides a monoclonal antibody (mAb). The term "antibody" relates to an antibody characterized as being specifically directed against CCL17 or its receptors or any functional derivative thereof. It could also be an antigen-binding fragment thereof, e.g. the F (ab')₂, F (ab) or single chain Fv type, or any type of recombinant antibody derived thereof. The antibodies have no cross-reactivity to others proteins.

The monoclonal antibodies can for instance be produced by any hybridoma liable to be formed according to classical methods from splenic cells of an animal, particularly of a mouse or rat immunized against CCL17 or its receptors or any functional derivative thereof, and of cells of a myeloma cell line, and to be selected by the ability of the hybridoma to produce the monoclonal antibodies recognizing CCL17 or its receptors or any functional derivative thereof which have been initially used for the immunization of the animals.

Research grade monoclonal Antibodies against CCL17, CCR4, and/or CCR8 are already commercially available, e.g. antibodies against CCL17 are CCL17/TARC (Mouse) antibody (Clone 110904)/ Rat IgG2A, TARC / CCL17 (Human) antibody (ABIN116235), TARC /CCL17 (Human) antibody (ABIN116236), TARC / CCL17 (Human) antibody (AB-IN116237), TARC / CCL17 (Human) antibody. (ABIN116238), chemokine (C-C-motif) ligand 17 (CCL17) (Human) antibody (ABIN171147), Small Inducible Cytokine Subfamily A (CYS-CYS)/ Member 17 (SCYA17) (CCL17) (Human) antibody (ABIN242265), Small Inducible Cytokine Subfamily A (CYS-CYS)/ Member 17 (SCYA17) (CCL17) (Human) antibody (ABIN296502), Small Inducible Cytokine Subfamily A (CYS-CYS)/ Member 17 (SCYA17) (CCL17) (Human) antibody (ABIN346404), Small Inducible Cytokine Subfamily A (CYS-CYS)/ Member 17 (SCYA17) (CCL17) (Human) antibody (ABIN215494), and Small Inducible Cytokine Subfamily A (CYS-CYS)/ Member 17 (SCYA17) (CCL17) (Human) antibody (ABIN334140).

Examples for research grade antibodies against CCR4 are CD194 (CCR4) (Human) Alexa Fluor 647 antibody (ABIN311834), CD194 (CCR4) (Human) PerCP/Cy5.5 antibody (AB-IN313319), CCR4 (Human) antibody (ABIN130991), CCR4 (Human) antibody (AB-IN223139), CCR4 (Mouse) antibody (ABIN347951), CCR4 (Mouse) antibody (AB-IN147324), CCR4 (Human) antibody (ABIN223485), CCR4 (Human) antibody (AB-IN179723), CCR4 (Mouse) antibody (ABIN285706), CD194 (CCR4) (Mouse) APC antibody (ABIN312092), CD194 (CCR4) (Mouse) APC antibody (ABIN312093), CD194 (CCR4) (Mouse) antibody (ABIN313846), CD194 (CCR4) (Mouse) antibody (ABIN313847), CD194 (CCR4) (Mouse) PE antibody (ABIN313002), CD194 (CCR4) (Mouse) PE antibody (AB-IN313003), CD194 / CCR4 (Human) antibody (ABIN270933), CD194 / CCR4 (Human) antibody (ABIN127098), CD194 / CCR4 (Human) antibody (ABIN314890), CD194 / CCR4 (Human) antibody (ABIN314909) and CD194 (CCR4) (Mouse) Alexa Fluor 647 antibody (ABIN311919).

Further examples for research grade antibodies against CCR8 are CCR8 (EL) (Human) antibody (ABIN274565), CCR8 (EL) (Human) antibody (ABIN274859), CCR8 (Human) antibody (ABIN131382), CCR8 (Human) antibody (ABIN149639), CCR8 antibody (ABIN142980), CCR8 EL antibody (ABIN289289), CCR8 (Human) antibody (ABIN223555), CCR8 (Mouse) antibody (ABIN347952), CCR8 (Human) antibody (ABIN281143), CCR8 (Human) antibody (ABIN281144), CDw198 / CCR8 (Human) antibody (ABIN260585), CDw198 / CCR8 (Human) antibody (ABIN264547), CDw198 (CCR8) (Mouse) antibody (ABIN313927), CDw198 (CCR8) (Mouse) antibody (ABIN313928), CDw198 / CCR8 (Human) antibody (AB-IN184212), CDw198 / CCR8 (Human) antibody (ABIN267994), CDw198 / CCR8 (Human) antibody (ABIN319016), CDw198 / CCR8 (Human) antibody (ABIN251774), CDw198 /CCR8 (Human) antibody (ABIN314886) and CDw198 (CCR8) (Mouse) Alexa Fluor 647 Antibody (ABIN311959).

These antibodies have been developed for research use and are thus not suitable for theraeutic use, as they are, in most cases, derived from rodents, like mice or rabbits. Administration to humans would evoke an immune response (e.g., HAMA response against murine mAbs).

However, a well-equipped toolbox for creating mAbs which are suitable for therapeutic use is today availabe for the skilled person. Therefore, once a target is known it is relatively easy for the skilled person to develop a therapeutic antibody against that target which is suitable for human use. Furthermore, modifying a murine monoclonal antibody, as one of the antibodies described above, in such way that they may be used for therapeutic purposes are routine methods in molecular engineering. For example, the constant chains are replaced by human IgG sequences ("chimerization"), or all sequences except of the CDR regions of the variable chains are replaced by human IgG sequences ("humanization"). Such approaches are well established today, and most therapeutic antibodies om the market today have in such way been derived from murine monoclonal antibodies. Some of the methods used for such purpose are described below.

Thus, in another preferred embodiment monoclonal antibody is at least one selected from the group consisting of murine, chimeric, humanized and/or human mAb; IgG, F(ab)2, Fab, and/or scFv; and/or modified antibody format.

Methods for the production and/or selection of chimeric, humanized and/or human mAbs are known in the art. For example, US6331415 by Genentech describes the production of Chimeric Antibodies, while US6548640 by Medical Research Council describes CDR grafting techniques and US5859205 by Celltech describes the production of Humanized Antibodies. In vitro antibody libraries are, among others, disclosed in US6300064 by MorphoSys and US6248516 by MRC/Scripps/Stratagene. Phage Display techniques are for example disclosed in US5223409 by Dyax. Transgenic mammal platforms are for example described in US200302048621 by TaconicArtemis.

IgG, F(ab)2, Fab, and scFv are known to the skilled person from respective literature.

Modified antibody formats are for example bi- or trispecific antibody constructs, shark or camelid antibodies, scFV modifications, Nano-, Mini or Diabodies, Antibody-based fusion proteins, antibody-drug conjugates, immunotoxins and the like.

In another embodiments the agent is at least
● a non-antibody-based target binding entity.
● A small molecular drug inhibiting the binding reaction between a CCL17 cytokine and a CCL17 receptor, and/or
● a pan-receptor antagonist.

Non-antibody-based target binding entities are for example Ankyrin Repeat Proteins, C-Type Lectins, A-domain proteins of Staphylococcus aureus, Transferrins, Lipocalins, Fibronectin, Kunitz domain protease inhibitors, Gamma crystallin, Cysteine knots or knottins, thioredoxin A scaffolds, nucleic acid aptamers, or Artificial Antibodies produced by molecular imprinting of polymers.

A small molecular drug is by definition not a polymer. The term is restricted to a molecule that also binds with high affinity to a biopolymer such as protein, nucleic acid, or polysaccharide and in addition alters the activity or function of the biopolymer, here CCL17 or its receptors. The upper molecular weight limit for a small molecule is approximately 800 Daltons which allows for the possibility rapid diffuse across cell membranes so that they can reach intracellular sites of action. In addition, this molecular weight cutoff is necessary but insufficient condition for oral bioavailability.

A pan-receptor antagonist can for example comprise at least part of the peptide sequence of CCL17, thus blocking the respective receptor without eliciting a receptor response. It can for example consist of an N-terminally modified CCL17 molecule.

The use of the agent for the preparation of a medicament is yet another aspect of the invention.

The term "medicament" refers to a composition or pharmaceutical composition comprising one or more agents according to the first aspect of the invention as defined herein before. The medicament may optionally comprise an acceptable carrier, excipient and/or auxiliary agent.

The medicament may optionally comprise a pharmaceutically acceptable carrier. Pharmaceutical formulations are well known and pharmaceutical compositions may be routinely formulated by one having ordinary skill in the art. The medicament can be formulated as a solution, suspension, emulsion, or lyophilized powder in association with a pharmaceutically acceptable vehicle. Examples of such vehicles are water, saline, Ringer's solution, dextrose solution, and human serum album in. Liposomes and non-aqueous vehicles such as fixed oils may also be used. The vehicle or lyophilized powder may contain additives that maintain in isotonicity (e.g. sodium chloride, mannitol) and chemical stability (e.g. buffers and preservatives). The composition is sterilized by commonly used techniques.

The medicament may be administrated by any means that enables the active agent to reach the agents site of action in the body of a mammal. The medicament may be administered parentally, i.e. intravenous (i.v.), subcutaneous (s.c), intramuscular

Dosage varies depending upon known factors such as the pharmacodynamic characteristics of the particular agent, and its mode and route of administration; age, health, and weight of the recipient; nature and extent of symptoms, kind of concurrent treatment, frequency of treatment, and the effect desired.

### Brief description of the examples and drawings

Additional details, features, characteristics and advantages of the object of the invention are disclosed in the subclaims, and the following description of the respective figures and examples, which, in an exemplary fashion, show preferred embodiments of the present invention. However, these drawings should by no means be understood as to limit the scope of the invention.

### Methods

### BMDC culture

DCs were cultured from bone marrow-derived Flt3⁺ progenitor cells according to techniques known in the art. Femurs and tibias were aseptically removed from donor mice, marrow cavities were flushed and bone marrow cell suspensions seeded at 2x10⁶ cells/ml in supplemented RPMI-1640 medium. After 3 days in culture, cells were subjected to Ficoll-Hypaque density gradient centrifugation (density, 1.077 g/ml; Eurobio). After 7 days, differentiation of Flt3⁺ progenitor cells into DCs was induced with 250 U/ml GM-CSF alone.

### Mouse models and BM transplantation

CD11c-EYFP mice were provided by Dr. M. Nussenzweig, *CCR4^{-l-}* mice were from Dr. I. Förster. *CCL17*^{E/E} mice were crossed with *Apoe^{-l-}* mice (all C57BL/6J background). Female *Apoe^{-l-}, CCL17*^{*E*/}*⁺Apoe^{-l-}*and *CCL17*^{*E*/}*^{E}Apoe^{-l-}* littermates were fed a normal chow. At 8 weeks of age, mice were placed on atherogenic diet (21% fat, 0.15% cholesterol, Altromin). BM transplantations were performed using techniques well known in the art. Briefly, BM-cells (5×10⁶ in PBS) from donor mice were administered to recipient mice by intravenous tail vein injection 24 h after an ablative dose of whole-body irradiation (2x6.5 Gy). Some 8 week-old *Apoe^{-l-}* mice were placed on atherogenic diet and after 4 weeks were treated intraperitoneally with an anti-CCL17 antibody (200 µg/injection, 3 times/week, rat IgG2a, R&D Systems) or corresponding isotype control antibody (200 µg/injection, 3 times/week, rat IgG2a, BioXcell) for additional 4 weeks of diet. Some 8 week-old *Apoe^{-l-}* mice were treated intraperitoneally with 2 doses of an anti-mouse CD4-depleting antibody (300 µg/injection, GK1.5, BioLegend) at 7 and 4 days before adoptive transfer of 5×10⁶ CD4⁺ T cells by tail vein injection. Donor CD4⁺ or OT-II T cells were isolated from secondary lymphoid organs of *Apoe^{-l-}* and *CCL17*^{*E*/}*^{E} Apoe^{-l-}* mice by negative immunomagnetic separation using CD4⁺ T-cell isolation kit II (Miltenyi). *CCL17*^{*E*/}*⁺ Apoe^{-l-}* and *CCL17*^{*E*/}*^{E} Apoe^{-l-}* littermates were fed a high-fat diet and intraperitoneally injected with an anti-CD25 depleting antibody or isotype control (clone PC61, Bioceros). For vaccination studies, cultured BMDCs were left untreated or pulsed with oxLDL for 1 h and 0.5×10⁶ sorted EGFP⁺ *CCL17*^{*E*/+} *Apoe^{-l-}* or *CCLI7*^{*E*/}*^{E}Apoe^{-l-}* littermates BMDCs were subcutaneously transferred to 8 week-old *Apoe^{-l-}* mice on day 0, 5, 7 and 35 after the initiation of high-fat diet. Animal experiments were approved by local authorities and complied with German animal protection law.

### Flow cytometry and cell sorting

Flow cytometry and cell sorting were carried out according to protocols known in the art, for details of the staining for flow cytometric analysis, intracellular labeling, surface staining and FACS analysis of tissues please see "Weber et. al (20XX)".

### T-cell proliferation in vitro and in vivo

CD4⁺ T cells were isolated from the spleen of OT-II mice by CD4 microbeads (Miltenyi), and labeled with CFSE (Fluka). Sorted DCs were pulsed for 30 min with 1 µg/ml with OVA₃₂₃-₃₃₉ peptide (ISQAVHAAHAEINEAGR, OVA-2, ANASPEC) for MHC class II-restricted T-cell stimulation. Untreated DCs were used as control. A total of 3×10⁴ OT-II T cells were co-cultured with 10⁴ DCs in 200 µl of medium containing GM-CSF in a 96-well flat-bottom microtiter plate for 72 h at 37°C. T-cell proliferation was analyzed by CFSE dye dilution after 3 days by flow cytometry. For *in vivo* studies, 5×10⁶ CD4⁺ CFSE-labelled OT-II cells were injected into the tail vein of C57BL/6 recipient mice. One day later, 5×10⁶ untreated or OVA-2 peptide-pulsed DCs were injected into the footpad. After 3 days, single-cell suspensions were prepared from popliteal lymph nodes of recipient mice to analyze T-cell proliferation.

### Oligo-Microarray

Total RNA isolation, assay set-up and detection were carried out according to protocols known in the art. Moreover data have been uploaded to the GEO database (accession: http://www.ncbi.nlm.nih.gov/geo/query/acc.cgi?token=hrwxvcmeswcygdc&acc=GSE 13642 http://www.ncbi.nlm.nih.gov/geo/query/acc.cgi?token=pxuppqiugokogbg&acc=GSE13592).

### Quantitative real time-polymerase chain reaction (PCR) and ELISA

RNA was isolated from lymph nodes by TRIzol (Invitrogen). cDNA was reverse transcribed from 2 µg of DNAse-treated total RNA (Promega). Real time-PCR analysis was performed using SYBRGreen (Clontech) and specific primer pairs (Sigma) according to manufacturer's protocols: Amplification (45 cycles, annealing at 58°C) was performed in duplicate using a MJ Research Opticon 2 (Biozym, Germany).

| **Name** | **Forward Primer 5'-3'** | **SEQ ID** | **Reverse Primer 5'-3'** | **SEQ ID** |
|---|---|---|---|---|
| *Il-2:* | CCTGAGCAGGATGGAGAATT | 1 | TCCAGAACATGCCGCAGAG | 2 |
| *Ifn-γ:* | TGGCTCTGCAGGATTTTCAT | 3 | TCAAGTGGCATAGATGTGGA | 4 |
| *Il-4* | CAACGAAGAACACCACAGAGAG | 5 | ATGAATCCAGGCATCGAAAAGC | 6 |
| *Il-10* | TGCACTACCAAAGCCAGAAGG | 7 | TGGGAAGTGGGTGCAGTTATTG | 8 |
| *Il-12a* | CTGTGGTCTTCAGCAGGTTT | 9 | CACGCTACCTCCTCTTTTTG | 10 |
| *Il-17a:* | CCTAAGAAACCCCCACGTTT | 11 | CAGTTTGGGACCCCTTTACA | 12 |
| *Foxp3* | TT GGTTTACTCGCATGTTCG | 13 | AGGGATTGGAGCAGCACTTGTTG | 14 |
| *Tgf-β* | TG ACGTCACTGGAGTTGTAC | 15 | GGTTCATGTCATGGATGGTG | 16 |
| *CCR4* | CTGACA TGTCCAGCTCCTCT | 17 | GAGCTTGTTAACGCCATGTT | 18 |
| *CCR8* | GCTGTCTATGC CATCAAGGT | 19 | CTTCAGAGGCCACTTGGTAA | 20 |
| *Gapdh* | CCATCACCATCTTC CAGGAG | 21 | GTGGTTCACACCCATCACAA | 22 |

### T-cell polarization assays

Effector Th1 or Th2 cells were generated by *in vitro* polarization of CD4⁺ cells as described. CD4⁺ spleen and LN cells were obtained by CD4 microbeads (Miltenyi). 5x10⁵ CD4⁺ T cells were cultured in 24-well tissue culture plates in 1 ml DC-medium in the presence of 3 µg/ml anti-CD3e (clone: 145-2C11) and 6 µg/ml anti-CD28 (clone: 37.51, eBioscience). Th1 cultures were supplemented with 50 U/ml recombinant murine IL-2 (rmIL-2), Th2 cultures contained rmIL-4 (500 U/ml) and purified rat anti-mouse IFN-γ antibody (10 µg/ml; all BD Biosciences) and Th17 cultures contained TGF-β (5ng/mL), Il-6 (20ng/mL) and anti-IFN-γ. (10 µg/ml). mCCL17 was added at a concentration of 50µg/mL. Tregs. After 3 days of culture, effector cells were stimulated in RPMI 1640/10% FCS containing GolgiStop (4 µl/ml; BD) and brefeldin (10 µg/ml; all from Sigma-Aldrich) at 37°C for 4 h, Samples were fixed by incubation in 100 µl of PBS/4% paraformaldehyde for 20 min, and cells were permeabilized by addition of 200 µl permeabilization buffer (PB: PBS/1% saponin) for 10 min. Samples were stained for 30 min at 4°C in PB containing appropriate anti-cytokine antibodies. After washing twice with PB, samples were resuspended in Hanks complete and analyzed using flow cytometry.

### Adoptive transfer of CFSE⁺ T cells

CD4⁺CD25⁺ or CD4⁺CD25⁻ T cells were sorted from blood, LNs and spleens, labeled with CFSE, and transferred intravenously. After 12 days, mice were sacrificed and LNs isolated and processed for flow cytometry. Transferred cells were detected by CFSE and CD4 staining.

### Quantification and immunhistochemical analysis of atherosclerosis

The extent of atherosclerosis was assessed in aortic roots and on thoracoabdominal aortas by staining for lipid depositions with oil-red-O and quantified by computerized image analysis (Diskus Software, Hilgers, Königswinter) and Leica Qwin Imaging software (Leica Ltd, Cambridge, UK). Briefly, atherosclerotic lesions were measured in 5-µm transversal sections through the heart and the aortic roots. The thoracoabdominal aorta was opened longitudinally, and the percentage of lipid deposition was calculated by dividing the stained area by the total thoracoabdominal aortic surface. The relative content of macrophages, CD3⁺ T cells, and smooth muscle cells was determined by mAb staining for MOMA-2 (MCA519), CD3 (MCA1477, both Serotec, Kidlington, Oxford, UK), and smoothelin (N-15, Santa Cruz) and detection with Cy3-conjugated antibody (Jackson ImmunoResearch, West Grove, PA). Nuclei were counter-stained by 4',6-Diamidino-2-phenylindol (DAPI). Images were recorded with a Leica DMLB fluorescence microscope and CCD camera. Antibodies to oxLDL were measured by chemiluminescent ELISA. Briefly, sera were diluted 1:100 and binding of IgGI to malondialdeyhde-modified LDL was measured using AP-conjugated rat-anti-mouse IgG1, followed by detection with LumiPhos.

### In vivo DC homing

The migration of CD11c⁺ vascular DCs, sorted from tissue digested aortas of CD11-EYFP mice (5x10⁵) , and sorted EGFP⁺*CCL17*^{*E*/+} and EGFP⁺*CCL17*^{*E*/*E*} DCs (5x10⁶) was assessed after injection of into the footpad of recipient C57B1/6 mice after 18 h by flow cytometry of popliteal lymph node cells. For T-cell homing assays, isolated and CFSE-labeled CD4⁺ T cells (5×10⁵) were injected intravenously into untreated C57B1/6 mice or 2 h after intraperitoneal administration of CCL17 (100 µg in sterile PBS). Recruitment to peripheral and para-aortic lymph nodes was assessed after 2 h or 4 days by FACS analysis.

### Examples

### I) Preliminary experiments

In order to study the function of CCL17 in atherosclerosis progression a CCL17-knock-out /EGFP knock-in reporter mouse, which allows for the localization of mature, CCL17 expressing DCs, was crossed with the Apo -/- mouse model, which permits study of a spontaneous atherosclerotic lesions and atherosclerosis progression. Thus in the resulting *CCL17* ^{*E*/}*^{E} Apo -* /- mice and *CCL17* ^{*E*/+} *Apo* -/- mice the localization and function of CCL117 positive, mature DCs in native and diet-induced atherosclerosis could be studied.

### II) Experiments showing that CCL17⁺ DCs control T-cell proliferation and polarization

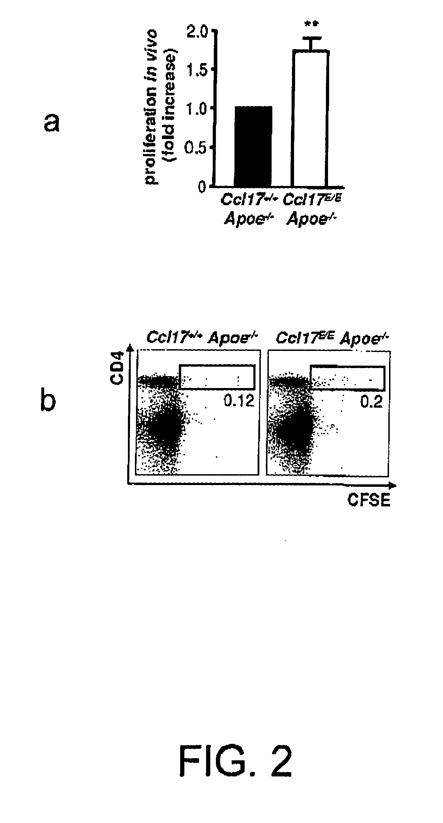

In summary these experiments demonstrated that
- antigen-specific T-cell proliferation induced by OVA-2-pulsed EGFP+*CCL17*^{*E*/*E*} DCs *in vivo* was more pronounced, as compared to EGFP⁺ *CCL17*^{*E*/+} DCs.
- exposure to EGFP⁺*CCL17*^{*E*/+} but not EGFP⁺*CCL17*^{*E*/*E*} DCs led to an up-regulation of the transcription factors *NF-κB, Stat1, Stat4, Socs3, Gata3* and the Th1 cytokine *Ifn-γ.*
- a Treg-dependent suppression of inflammatory cytokines may account for the differences in Th1 and Th17-cell polarization in the co-culture experiments with EGFP⁺ *CCL17*^{*E*/*E*} DCs.
- the cytokine environment in the vicinity of CCL17⁺ DCs or within the immunological synapse seems to critically shape the responses in interacting T-cells, suppressing Treg polarization, while instigating and sustaining inflammatory Th1 and Th17 responses.
- an increased frequency of foxp3⁺CD25⁺ Tregs was observed in *CCL17*^{*E*/}*^{E}Apoe^{-l-}* mice compared to *CCL17^{ElE} Apoe^{-l-}* mice, indicating an enhanced conversion from naïve cells (Fig. 1).
- as compared to *CCLI7*^{*+*/}*⁺Apoe^{-l-}* mice, an increased proliferation of CFSE⁺CD4⁺CD25⁺ Tregs was observed in recipient *CCL17*^{*E*/}*^{E} Apoe^{-l-}* mice (Fig. 2) supporting a role of CCL17⁺ DCs in suppressing both polarization and homeostatic proliferation of Tregs.

### III) Experiments showing that CCL17-expressing DCs accumulate in atherosclerotic lesions

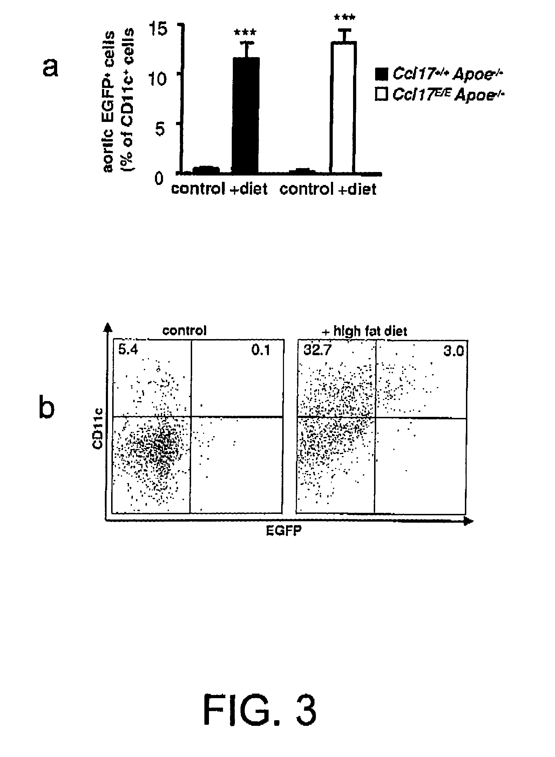

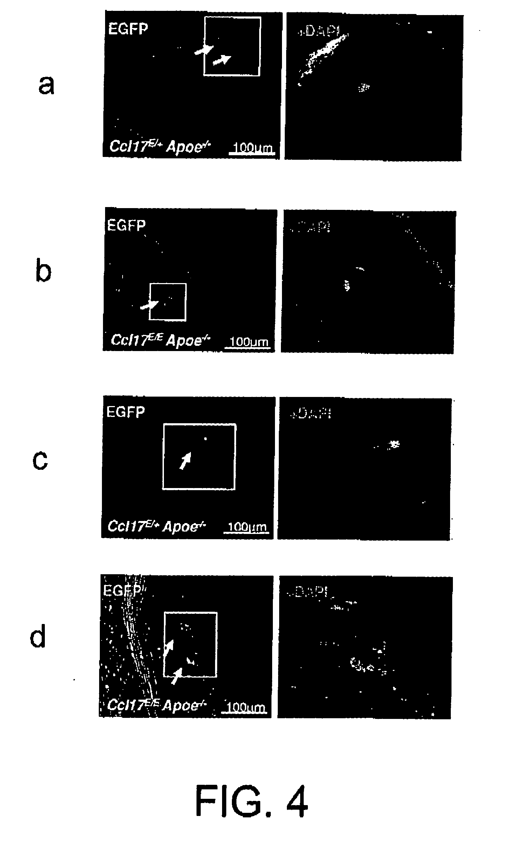

Given the presence of CCL17⁺ DCs in various organs it was tested whether CCL17⁺ DCs locally accumulate in atherosclerotic lesions. FACS analysis (Fig. 3) and immunofluorescence (Fig. 4) of enzymatically digested aortas CCL17^{E/+} Apoe^{-/-} or CCL17^{E/E} Apoe^{-/-} mice with diet-induced atherosclerosis demonstrated that CD11c⁺EGFP⁺ DCs were increased in aortic lesions.

Moreover CCL17 transcripts were increased in Apoe^{-/-} aortas with advanced lesions (3.8±0.8-fold over healthy aortas, n=4, P<0.01). Notably, CCL17⁺ DCs were detectable in advanced aortic root plaques, adventitia and lymph nodes of chimeric Apoe^{-/-} mice reconstituted with CCL17^{E/+} Apoe^{-/-} BM suggests a continuous influx of BMDCs or their precursors during lesion progression.

### IV) Experiments showing that deficiency of CCL17 reduces atherosclerosis in Apoe^{-/}⁻ mice

### Example IVa

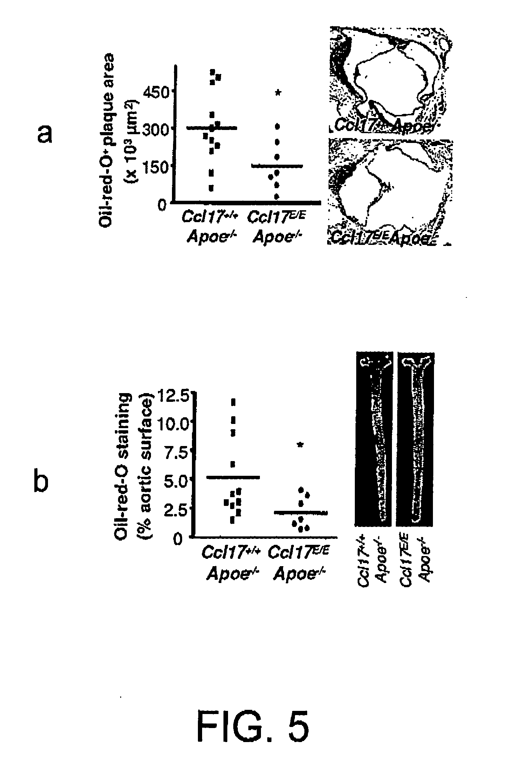

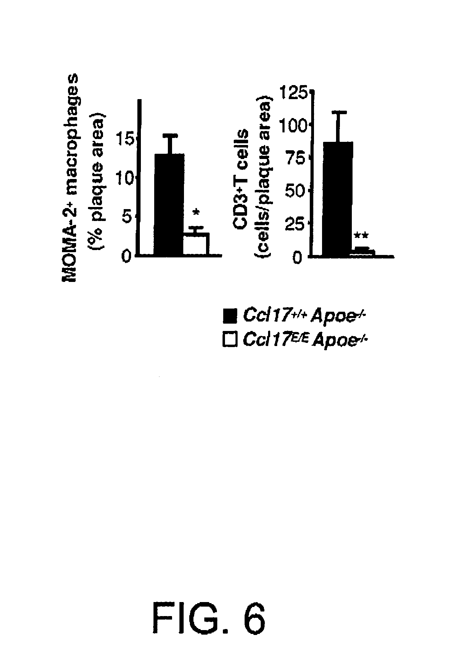

The role of CCL17⁺ DCs in atherosclerotic lesion formation was investigated. Compared with *CCL17* ^{+/+}*Apoe^{-l-}* controls, *CCL17*^{E/E} *Apoe*^{*-*/*-*} mice deficient in CCL17 displayed markedly reduced plaque formation in the aortic root and in the thoracoabdominal aorta after 6 months of normal chow (Fig. 5). This was associated with a decreased macrophage content (Fig. 6) and increased smooth muscle cell content (9.0±2.0% *vs* 1.9±0.6% smoothelin⁺ plaque area, P<0.005), indicating a shift towards a more stable plaque phenotype in *CCL17*^{E/E} *Apoe^{-l-}* mice. Although the frequency of lesional EGFP⁺ DCs did not differ (data not shown), CD3⁺ T cells were reduced in *CCL17*^{E/E} *Apoe*^{*-*/}*⁻ vs CCL17* ^{E/+} *Apoe*^{*-*/*-*} plaques (Fig. 6), implicating CCL17 in T-cell attraction or retention.

### Example IVb

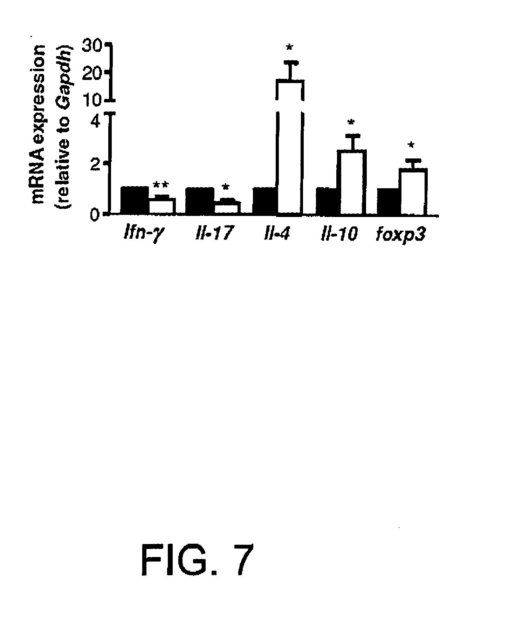

Real-time PCR analyses of lymph node cells collected from atherosclerotic *CCL17*^{E/E} *Apoe*^{*-*/*-*} mice displayed reduced expression of *Ifn-γ* and *Il-17* mRNA but increased levels of *Il-4, Il-10 and foxp3* mRNA, relative to *CCL17*^{+/+}*Apoe^{-l-}mice* (Fig. 7).

### Example IVc

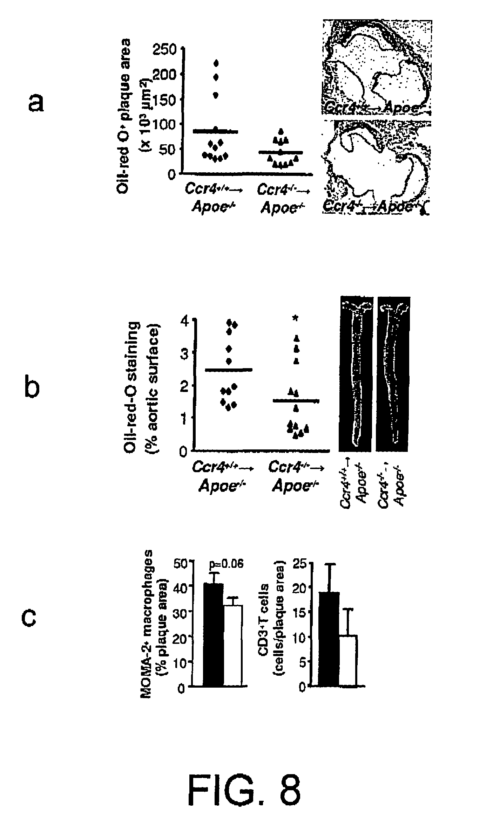

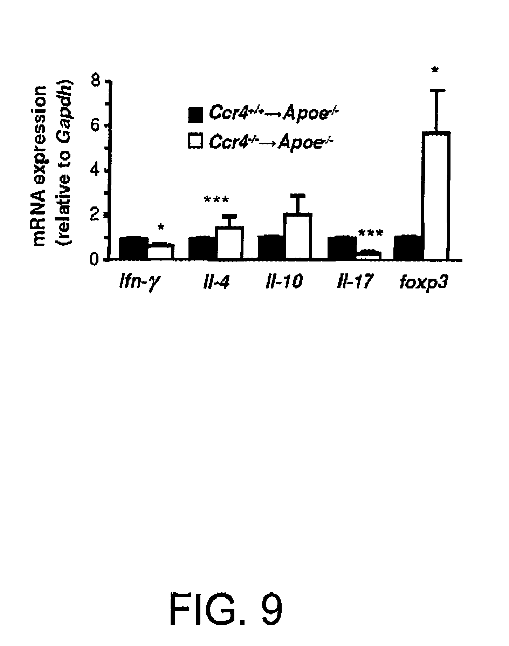

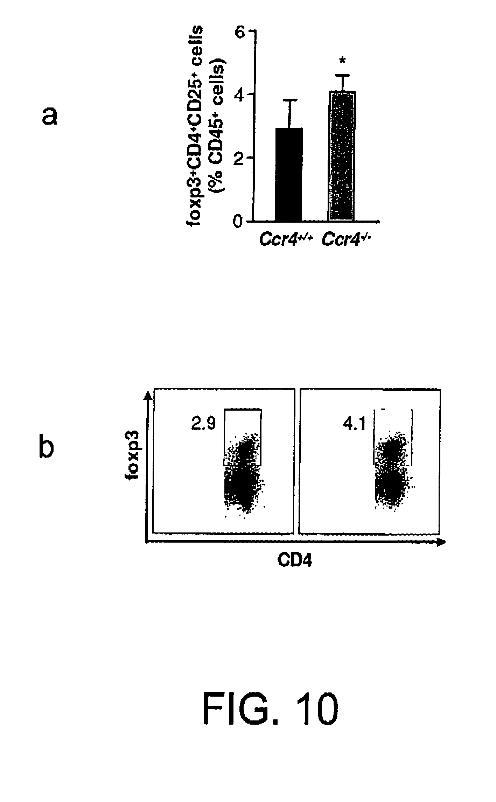

Both CCR4 and CCR8 have been shown to function as receptors for CCL17 and to be expressed on Th1, Th2 and Tregs. The mRNA expression of CCR4 and CCR8 and CCR4 surface expression were detectable on polarized Th1, Th2, Th17 cells and Tregs, as assessed by real-time PCR and flow cytometry (data not shown). Notably, lesion formation and content of macrophages and CD3+ T cells was reduced in the aortic root and aorta of Apoe-/- mice reconstituted with CCR4-deficient BM *(CCR4-*/*-→Apoe-*/*-),* compared with *CCR4*+/+→*Apoe-*/*-* mice (Fig. 8 Fig. 4g,h). This was accompanied by reduced levels of *Ifn-γ* and *Il-17* mRNA and elevated *Il-4, Il-10* and *foxp3* mRNA (Fig. 9 Fig 4i), implying that functions of CCL17 in atherosclerosis are largely CCR4-mediated. An expansion of foxp3+CD25+CD4+ Tregs was also observed in *CCR4-*/*-* when compared to *CCR4*+/+ mice (Fig. 10 Fig. 4j). Thus, DC-derived CCL17, likely via autocrine CCR4 signaling, promotes atherosclerosis and is required for suppressing Treg responses, skewing the immune response towards a Th1/Th17-1ike polarization.

### Example IVd

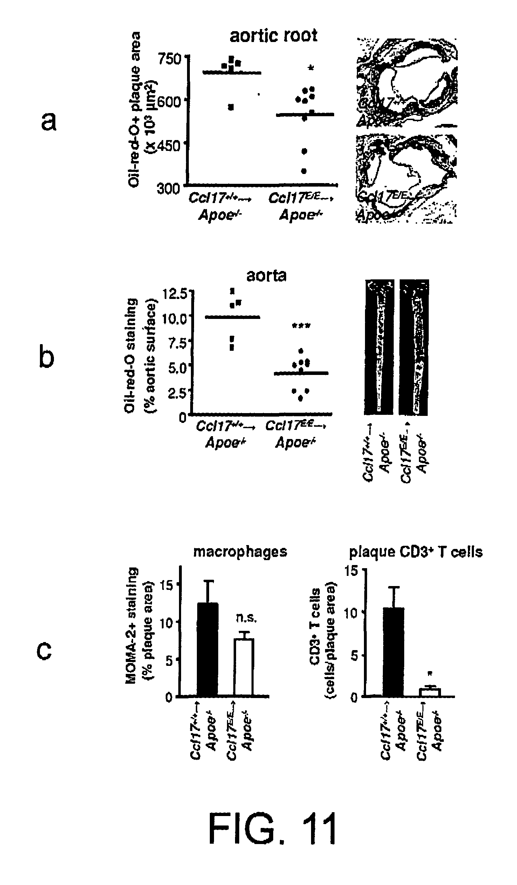

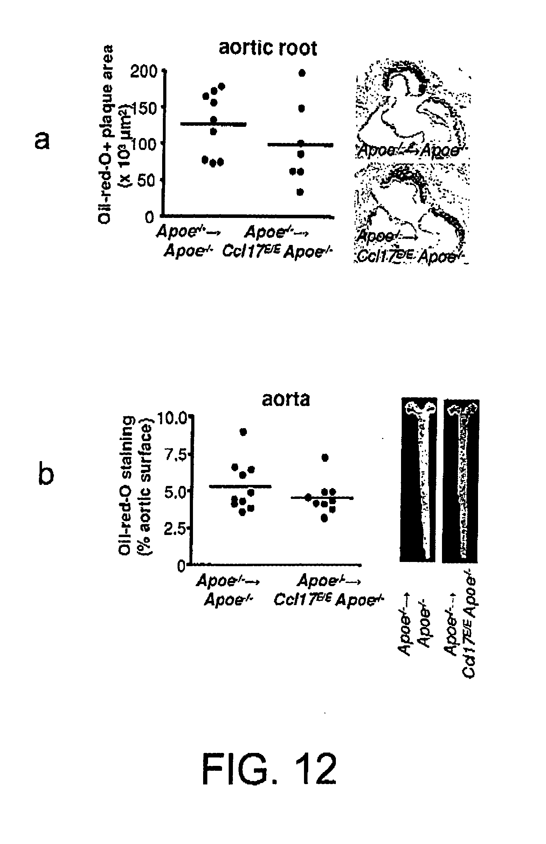

To scrutinize the contribution of CCL17 to lesion progression, *Apoe^{-l-}* mice bearing established atherosclerotic lesions after 12 weeks of high-fat diet were reconstituted with *CCL17*^{+/+}*Apoe^{-l-}* or *CCL17*^{E/E}*Apoe^{-l-}* BM. In *CCL17*^{E/E}*Apoe^{-l-}* recipients *(CCL17*^{E/E}*Apoe^{-l-}→Apoe*^{*-*/}*⁻),* lesion progression was markedly reduced in the aortic root and thoracoabdominal aorta after another 12 weeks of high-fat diet, and the content of macrophages and CD3⁺ T cells was diminished, compared with *CCL17*^{E/E}*Apoe^{-l-}→Apoe^{-l-}* mice (Fig. 11). In contrast, no significant differences in lesion formation were observed (Fig. 12), when comparing *CCL17*^{+/+}*Apoe^{-l-}* and *CCL17*^{E/E}*Apoe^{-l-}* mice reconstituted with wild-type *CCL17*^{+/+}*Apoe^{-l-}* BM, indicating that CCL17⁺ effector cells predominantly originate from BM.

### V) Experiments showing that T cells primed by CCL17⁺ DCs mediate atherogenesis

### Example Va

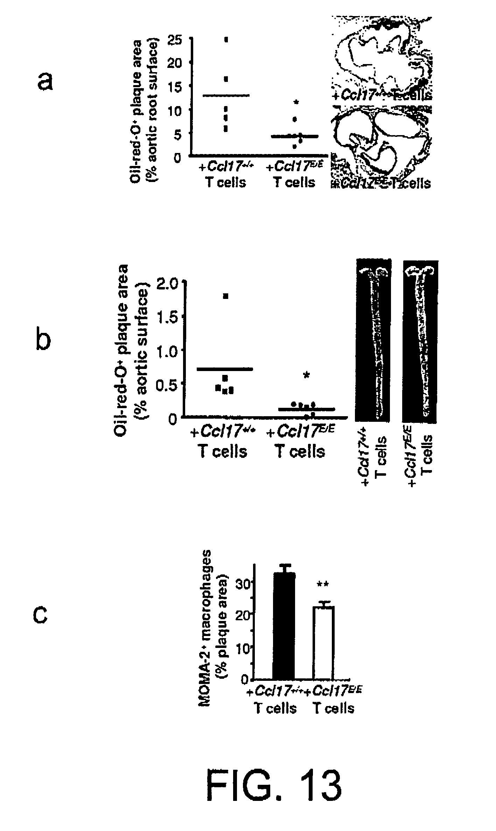

To address the effects of a CCL17-polarized T-cell help on atherosclerosis, CD4⁺ T cells were sorted from 6 months-old *CCL17*^{+/+}*Apoe^{-l-}* or *CCL17*^{E/E}*Apoe^{-l-}* mice and adoptively transferred into 8 week-old *Apoe*^{*-*/*-*} mice depleted from CD4⁺ cells. After 6 weeks of high-fat diet, lesion formation, and macrophage content were decreased in mice repleted with CD4⁺ T cells from *CCL17*^{*E*/}*^{E} Apoe*^{*-*/*-*} mice as compared to those receiving CD4⁺ T cells from *CCL17*^{+/+} *Apoe*^{*-*/*-*} mice (Fig. 13). Recapitulating the differences in T-cell polarization, *Apoe^{-l-}mice* repleted with T cells from *CCL17*^{E/E}*Apoe^{-l-}* mice displayed significantly lower *Ifn-γ* and *Il*-*17* transcripts (data not shown).

### Example Vb

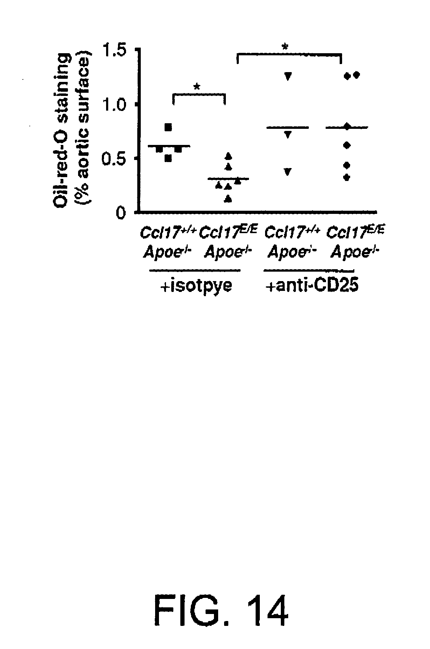

In a reciprocal approach, an antibody to CD25 was used to deplete Tregs in *CCL17*^{E/E}*Apoe^{-l-}* mice and *Apoe^{-l-}* controls. Depletion of CD25⁺ Tregs increased atherosclerotic lesion formation in controls and reversed the reduction of lesion formation in CCL17-deficient *Apoe*^{*-*/*-*} mice (Fig. 14). This implies that T cells imprinted by DCs in lymphoid organs can exert systemic effects on atherosclerosis, while protective effects of CCL17 deficiency is mediated by Tregs.

### VI) Experiments showing that Transferred CCL17⁺ DCs aggravate atherosclerosis

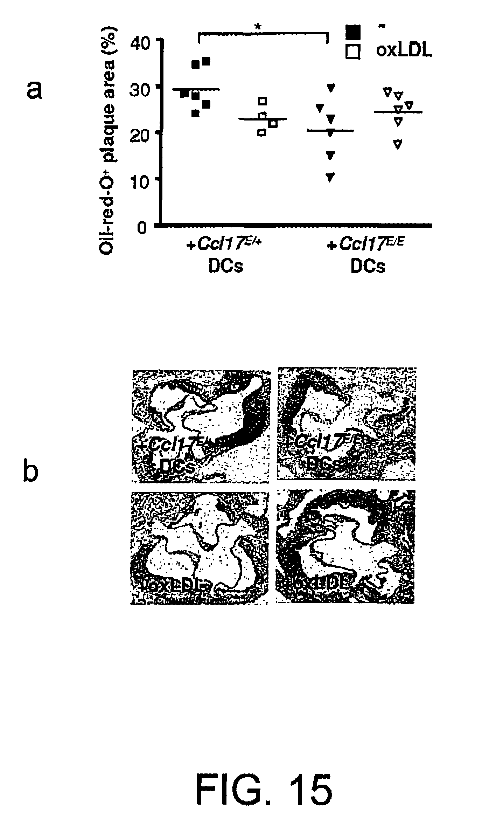

Intravenous transfer of LPS-activated DCs loaded with oxLDL has been shown to diminish collar-induced lesion formation in carotid arteries without affecting atherosclerosis in the aortic root. To directly assess effects of CCL17⁺ DCs on lesion formation, *Apoe^{-l-}* mice were subcutaneously injected with sorted unpulsed or oxLDL-loaded EGFP⁺*CCL17*^{E/+}*Apoe*^{*-*/-} or EGFP⁺*CCL17*^{E/E}*Apoe^{-l-}* BMDCs before and twice after starting high-fat diet. Notably, lesion formation in the aortic root was enhanced in mice transferred with unpulsed EGFP⁺*CCL17*^{E/+}*Apoe^{-l-} vs* EGFP⁺*CCL17*^{E/E}BMDCs, whereas *foxp3* mRNA was significantly up-regulated in lymph nodes of mice injected with EGFP⁺*CCL17*^{E/E} BMDCs (Fig. 15). In contrast, oxLDL-pulsed EGFP⁺CCL17^{E/+} or *CCL17*^{E/E} BMDCs did not alter lesion formation or *foxp3* expression. These data indicate that DCs account for the CCL17 effects and that an excess of CCL17⁺ DCs may suffice to repress Treg responses to enhance inflammation and atherosclerosis. In contrast, oxLDL loading may initiate alternative mechanisms to suppress or override CCL17-dependent effects.

### VII) Experiments showing CCL17 as a potential therapeutic target

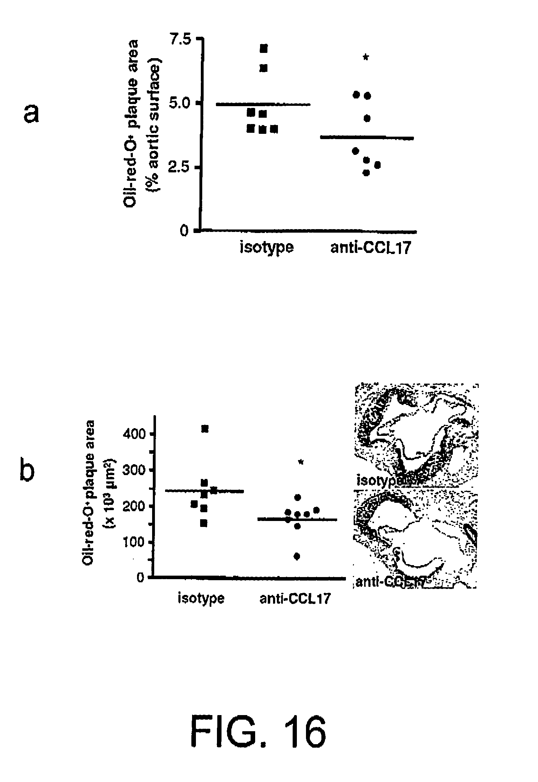

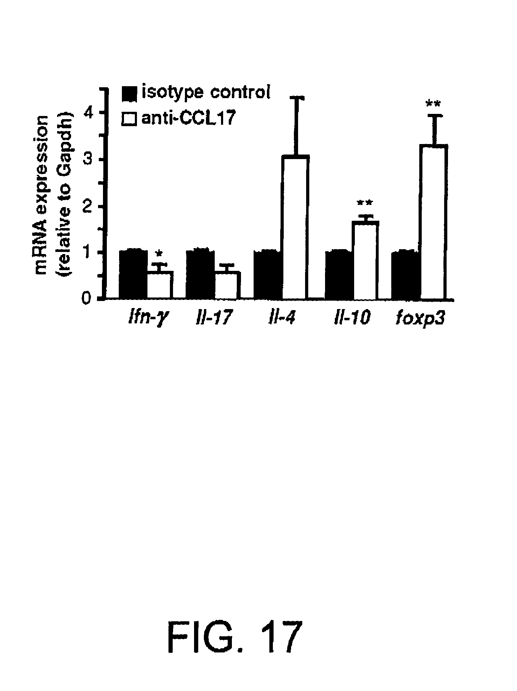

To verify CCL17 as a therapeutic target in atherosclerosis, a blocking antibody to CCL17 during disease progression was employed. Treatment was initiated after 4 weeks of diet in *Apoe^{-l-}* mice with early lesions and was continued for another 4 weeks of diet. Mice treated with anti- CCL17 displayed a retarded lesion formation and reduced macrophage content in the aortic root, compared with isotype control-treated *Apoe*^{*-*/*-*} mice (Fig. 16). This was accompanied by an expansion of foxP3⁺CD25⁺ T cells (Fig. 17), diminished expression of *Ifn-γ* and *Il-17* mRNA and increased levels of *Il-4, Il-10* and *foxp3* mRNA in lymph nodes of anti-CCL17-treated mice (Fig. 17). These data provide evidence that targeting CCL17 may constitute a feasible therapeutic approach for advanced atherosclerosis. Together, our results establish that effector functions exerted by CCL17⁺ DCs are central in sustaining inflammatory Th1/Th17-polarization, in controlling Treg responses and thereby in driving lesion formation.

### VIII) relevance of CCL17 for autoimmune diseases

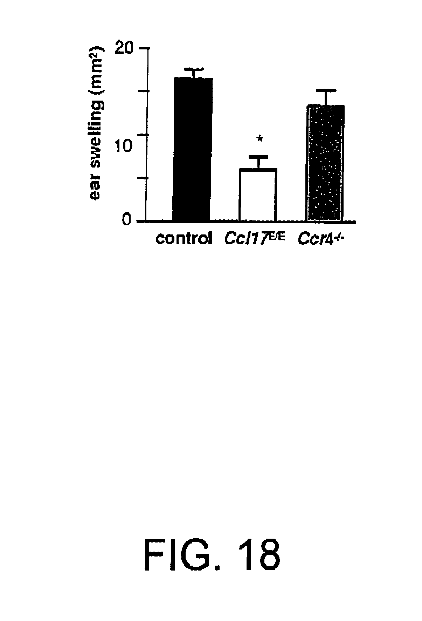

In a model for autoimmune diseases related to Th1-malfunction hypersensitivity dermatitis, or cutaneous hypersensitivity, inflammation as evident by ear thickness 24 h after challenge with DNFB was investigated. Contact hypersensitivity against dinitrofluorobenzene (DNFB, Sigma-Aldrich) was induced by application of 80 µl of 0.5% DNFB in acetone - olive oil (5:1) on the dorsal shaved skin of mice. After 5 days, mice were challenged by painting the dorsal and ventral side of both ears with 10 µl of 0.3% DNFB. Ear thickness was measured before and 24 h after challenge. The latter was markedly diminished in *CCLI7*^{*E*/*E*} mice as compared to wild-type controls and slightly but not significantly attenuated in *CCR4*^{*-*/*-*} mice (see Fig. 18).

### Discussion of the figures

Figure 1
   Fig. 1 shows that an increased frequency of foxp3⁺CD25⁺ Tregs was observed in *CCL17*^{*E*/}*^{E} Apoe* ^{*-*/*-*} mice compared to *CCL17*^{*E*/}*^{E} Apoe*^{*-*/*-*} mice, indicating an enhanced conversion, from naive cells. In the experiment CFSE-labeled CD4⁺CD25⁻ T cells were transferred to *CCL17*^{+/+}*Apoe^{-l-}* or *CCL17*^{E/E}*Apoe^{-l-}* mice and the frequency of CD4⁺CD25⁺ Tregs among CFSE⁺CD4⁺ T cells in lymph nodes was analyzed by flow cytometry after 12 days (a). Representative dot plots and percentages within gates are shown (b); n=10 per group, ****P*<0.001.
Figure 2
   Fig. 2 shows that as compared to *CCL17*+/+*Apoe-l-* mice, an increased proliferation of CFSE+CD4+CD25+ Tregs was observed in recipient *CCL17ElEApoe-l-* mice supporting a role of CCL 17+ DCs in suppressing both polarization and homeostatic proliferation of Tregs. In the experiment CFSE-labeled CD4+CD25+ T cells were transferred to *CCL17+*/*+Apoe-*/*-* or *CCL17E*/*EApoe-*/*-* mice and their maintenance analyzed by flow cytometry after 12 days (a). Representative dot plots and percentages within gates are shown (b); n=10 per group, * *P<0.001.
Figure 3
   Fig. 3 shows that CD11c⁺EGFP⁺ DCs were increased in aortic lesions of *CCL17*^{E/+}*Apoe*^{*-*/*-*} or *CCL17*^{E/E}*Apoe*^{*-*/*-*} mice with diet-induced atherosclerosis, as determined by FACS analysis of digested aortas. In detail quantification of EGFP⁺CD11c⁺ DCs in aortas of naïve *CCL17*^{*E*/+}*Apoe^{-l-}* and *CCL17*^{*E*/}*^{E}Apoe^{-l-}* mice or after high-fat diet feeding using enzymatic digestion and FACS analysis was carried out (a); representative dot blots and percentages within quadrants are shown (b). Bars represent mean±SEM; n=6 mice each, ****P*<0.0001.
Figure 4
   Fig. 4 shows that CD11c⁺EGFP⁺ DCs were increased in aortic lesions of *CCL17*^{E/+}*Apoe*^{*-*/*-*} or *CCL17*^{E/E} *Apoe*^{*-*/*-*} mice with diet-induced atherosclerosis, as determined by immunofluorescence. In detail the figures show EGFP⁺ DCs (indicated by arrows) in the aortic root of *CCL17*^{*E*/+}*Apoe^{-l-}* (a) and *CCL17*^{*E*/}*^{E}Apoe^{-l-}* (b) mice after 12 weeks of high-fat diet and EGFP⁺ DCs (indicated by arrows) in the aortic root of *CCL17*^{*E*/+}*Apoe^{-l-}* (c) and *CCL17*^{*E*/}*^{E} Apoe^{-l-}* (d) after 6 months of normal chow; cell nuclei are counter-stained by DAPI.
Figure 5
   Fig. 5 shows that compared with *CCL17*^{+/+}*Apoe*^{*-*/*-*} controls, *CCL17*^{E/E}*Apoe*^{*-*/*-*} mice deficient in CCL 17 displayed markedly reduced plaque formation in the aortic root and in the thoracoabdominal aorta after 6 months of normal. In the experiment atherosclerotic lesions were quantified in the aortic root and thoracoabdominal aorta after staining with oil-red O in *CCL17*^{+/+}*Apoe*^{*-*/*-*} and *CCL17*^{*E*/}*^{E} Apoe*^{*-*/*-*} mice on normal chow for 6 months; representative images of the aortic root (a) and the thoracoabdominal aorta (b) are shown.
Figure 6
   Fig. 6 shows that compared with *CCL17*^{+/+}*Apoe*^{*-*/*-*} controls, *CCL17*^{E/E}*Apoe^{-l-}* mice deficient in CCL 17 have a decreased macrophage content and increased smooth muscle cell content (9.0±2.0% *vs* 1.9±0.6% smoothelin⁺ plaque area, *P*<0.005), indicating a shift towards a more stable plaque phenotype in *CCL17*^{E/E} *Apoe^{-l-} mice.* Moreover although the frequency of lesional EGFP⁺ DCs did not differ (data not shown), CD3⁺ T cells were reduced in *CCL17*^{E/E} *Apoe*^{*-*/}*⁻vs CCL17*^{E/+}*Apoe*^{*-*/}*⁻plaques* implicating CCL17 in T-cell attraction or retention. In the experiment the relative frequency of MOMA-2⁺ macrophages and CD3⁺ T cells per plaque area were determined by quantitative immunofluorescence; **P*<0.05, ***P*<0.005.
Figure 7
   Fig. 7 shows that lymph node cells collected from atherosclerotic *CCL17*^{E/E} *Apoe*^{*-*/*-*} mice displayed reduced expression of *Ifn-γ* and *Il-17* mRNA but increased levels of *Il-4, Il-10* and *foxp3* mRNA, relative to *CCL17*^{+/+}*Apoe^{-l-}* mice. In the experiment mRNA expression and cytokine protein concentration was analyzed by real-time PCR in lymph nodes collected from *CCL17*^{+/+}*Apoe^{-l-}* and *CCL17*^{*E*/}*^{E} Apoe*^{*-*/*-*} mice fed a high-fat diet. n=6 mice per group; **P*<0.05, ***P*<0.01
Figure 8
   Fig. 8 shows that lesion formation and content of macrophages and CD3+ T cells was reduced in the aortic root and aorta of *Apoe*^{*-*/*-*} mice reconstituted with CCR4-deficient BM (*CCR4*^{*-*/}*⁻→Apoe^{-l-}*)*,* compared with *CCR4*+/+→*Apoe-*/*-* mice. In the experiment atherosclerotic lesions were quantified in the aortic root (a) and thoracoabdominal aorta (b) after staining with oil-red-O in *Apoe-*/*-* mice transplanted with CCR4+/+ or CCR4-/- bone marrow and fed a high-fat diet for 12 weeks; representative images are shown; *P<0.05. MOMA-2+ macrophages and CD3+ T cells per plaque area were determined by quantitative immunofluorescence (c).
Figure 9
   Fig. 9 shows that reduced lesion formation and content of macrophages and CD3+ T cells in the aortic root and aorta of *Apoe-*/*-* mice reconstituted with CCR4-deficient BM (*CCR4-*/*- →Apoe-*/*-*)*,* compared with *CCR4*+/+ *→Apoe-*/*-* mice as demonstrated in figure 8 above was accompanied by reduced levels of Ifn-γ and Il-17 mRNA and elevated Il-4, Il-10 and foxp3 mRNA , implying that functions of CCL17 in atherosclerosis are largely CCR4-mediated. In the experiment real-time PCR analysis of mRNA expression in lymph nodes of Apoe-/- mice transplanted with CCR4+/+ or CCR4-/- bone marrow and fed a high-fat diet for 12 week was carried out; *P<0.05, ***P<0.0005.
Figure 10
   Fig. 10 shows that an expansion of foxp3+CD25+CD4+ Tregs was also observed in *CCR4-*/*-* when compared to *CCR4*+/+ mice. Frequencies of Tregs were determined in lymph nodes of *CCR4*+/+ and *CCR4-*/*-* mice (a). Representative dot plots are shown (b); inserted numbers indicate percentages of CD45+ events; n=6 per group, *P<0.05. Bars represent mean±SEM.
Figure 11
   Fig. 11 shows that in *CCL17*^{E/E}*Apoe*^{*-*/*-*} recipients (*CCLI7*^{E/E}*Apoe*^{-/-}→*Apoe*^{-/-})*,* lesion progression was markedly reduced in the aortic root and thoracoabdominal aorta after another 12 weeks of high-fat diet, and the content of macrophages and CD3⁺ T cells was diminished, compared with *CCLI7*^{+/+}*Apoe*^{-/-}→*Apoe*^{-/-} mice. In the experiment *Apoe*^{*-*/*-*} mice fed a high-fat diet for 12 weeks were transplanted with either *CCLI7*^{+/+}*Apoe*^{-/-} (*CCLI7*^{+/+}*Apoe*^{-/-}→*Apoe*^{-/-}) or *CCLI7*^{*E*/}*^{E}Apoe*^{-/-} BM (*CCLI7*^{E/E}*Apoe*^{-/-}→*Apoe*^{-/-})*.* After an additional 12 weeks of high-fat diet, atherosclerotic lesions were quantified in the aortic root (a) and thoracoabdominal aorta (b) after staining with oil-red O; representative images are shown. The frequency of MOMA-2⁺ macrophages and CD3⁺ T cells per plaque area were determined by quantitative immunofluorescence (c). Bars represent mean±SEM. **P*<0.05, ****P*<0.001.
Figure 12
   Fig. 12 shows that no significant differences in lesion formation were observed when comparing *CCL17*^{+/+}*Apoe^{-l-}* and *CCL17*^{E/E}*Apoe^{-l-}* mice reconstituted with wild-type *CCL17*^{+/+}*Apoe^{-l-}* BM, indicating that CCL17⁺ effector cells predominantly originate from BM. In the experiment *CCL17*^{+/+}*Apoe^{-l-}* or *CCL17*^{E/E}*Apoe^{-l-}* mice were transplanted with *Apoe^{-l-}* bone marrow (*Apoe*^{*-*/}*⁻→Apoe*^{*-*/*-*} or *Apoe*^{*-*/}*⁻CCL17*^{E/E}*).* After 12 weeks of high-fat diet, atherosclerotic lesions were quantified in the aortic root (a) and thoracoabdominal aorta (b) after staining with oil-red-O; representative images are shown.
Figure 13
   Fig. 13 shows that after 6 weeks of high-fat diet, lesion formation and macrophage content were decreased in mice repleted with CD4⁺ T cells from *CCL17*^{E/E}*Apoe^{-l-}* mice as compared to those receiving CD4⁺ T cells from *CCL17*^{+/+} *Apoe*^{*-*/*-*} mice. In the experiment CD4⁺ T cells sorted from 6 months-old *CCL17*^{+/+}*Apoe^{-l-}* or *CCL17*^{E/E}*Apoe^{-l-}* mice on normal chow were adoptively transferred into 8 week-old *Apoe*^{*-*/*-*} mice depleted from CD4⁺ cells. After 6 weeks of high-fat diet, atherosclerotic lesions were quantified in the aortic root (a) and thoracoabdominal aorta (b) after oil-red-O staining; representative images are shown. The relative frequency of MOMA-2⁺ macrophages per plaque area was assessed by quantitative immunofluorescence (c). **P*<0.05, *P***<0.005.
Figure 14
   Fig. 14 shows that in a reciprocal approach to the one in Figure 15, where an antibody to CD25 was used to deplete Tregs in *CCL17*^{E/E}*Apoe^{-l-}* mice and *Apoe*^{*-*/*-*}controls, depletion of CD25⁺ Tregs increased atherosclerotic lesion formation in controls and reversed the reduction of lesion formation in CCL17-deficient *Apoe*^{*-*/*-*} mice. In the experiment atherosclerotic lesions were quantified in aortic roots and thoracoabdominal aortas after staining with oil-red-O in *CCL17*^{+/+}*Apoe^{-l-}* and *CCL17*^{E/E}*Apoe*^{*-*/}*⁻mice* fed a high-fat diet and treated with anti-CD25 depleting antibody or isotype control for 4 weeks. **P*<0.05
Figure 15
   Fig. 15 shows that lesion formation in the aortic root was enhanced in mice transferred with unpulsed EGFP⁺*CCL17*^{E/+}*Apoe^{-l-} vs* EGFP⁺*CCL17*^{E/E} BMDCs, whereas *foxp3* mRNA was significantly up-regulated in lymph nodes of mice injected with EGFP+ *CCL17*^{E/E} BMDCs. In the experiment atherosclerotic lesions were quantified in aortic roots aortas after oil-red-O staining in *Apoe*^{*-*/*-*} mice injected with unpulsed or oxLDL-pulsed EGFP⁺*CCL17*^{E/+} or EGFP⁺*CCL17*^{E/E} BMDCs and fed a high-fat diet for 8 weeks (a); representative images are shown (b). **P*<0.05 (f).
Figure 16
   Fig. 16 shows that neutralization of CCL 17 reduced the progression of atherosclerosis. In particular it was demonstrated that mice treated with anti- CCL17 displayed a retarded lesion formation and reduced macrophage content in the aortic root, compared with isotype control-treated *Apoe*^{*-*/*-*} mice. In the experiment *Apoe^{-l-}* mice fed a high-fat diet for 4 weeks were injected with anti-CCL17 blocking antibody or isotype control for an additional 4 weeks. Atherosclerotic lesions were quantified in the aortic root and aorta after oil-red-O staining (a); representative images are shown (b). **P*<0.05 (a,b).
Figure 17
   Fig. 17 shows that the retarded lesion formation and reduced macrophage content in mice treated with anti- CCL17 was accompanied by a diminished expression of *Ifn-γ* and *Il-17* mRNA and increased levels of *Il-4, Il-10* and *foxp3* mRNA in lymph nodes of anti-CCL17-treated mice. In the experiment real-time PCR analysis of mRNA levels in lymph nodes of *Apoe*^{*-*/*-*} mice fed a high-fat diet for 4 weeks and which were injected with anti-CCL17 blocking antibody or isotype control for an additional 4 weeks was carried out. **P*<0.05, ***P<*0.01. Bars represent mean±SEM.
Figure 18
   Fig. 18 shows, as a model for autoimmune diseases related to T helper cell Th1-malfunction, contact hypersensitivity in *CCL17*^{+/+}*, CCL17*^{E/E} or CCR4^{*-*/*-*} mice sensitized with 0.5% DNFB. After 5 days, sensitized mice were challenged with 0.3% DNFB. Ear thickness was measured 24 h after challenge; n=4-6 per group, **P*<0.01.-Bars represent mean±SEM.

### Disclaimer

To provide a comprehensive disclosure without unduly lengthening the specification, the applicant hereby incorporates by reference each of the patents and patent applications referenced above.

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. The invention's scope is defined in the following claims and the equivalents thereto. Furthermore, reference signs used in the description and claims do not limit the scope of the invention as claimed.

### References

Dardalhon V, Kom T, Kuchroo VK, Anderson AC. Role of Th1 and Th17 cells in organ-specific autoimmunity. J Autoimmun. 2008 Nov ;31(3): 252-6.
Perros F, Hoogsteden HC, Coyle AJ, Lambrecht BN, Hammad H.Blockade of CCR4 in a humanized model of asthma reveals a critical role for DC-derived CCL17 and CCL22 in attracting Th2 cells and inducing airway inflammation. Allergy. 2009 Jul; 64(7): 995-1002.

## Claims

1. Agent inhibiting the binding reaction between a CCL17 cytokine and a CCL17 receptor for use as a medicament.

2. Agent according to claim 1, wherein the medicament is a medicament for treating a T helper cell Th1-driven disease.

3. Agent according to claim 1 or 2, wherein the medicament is a medicament for treating an atherosclerotic disease with at least one pathologic condition selected from the group consisting of
● atherosclerosis
● myocardial infarction
● stroke
● peripheral vascular disease
● chronic kidney disease, and/or
● angina pectoris.

4. Agent according to claim 1 or 2, wherein the medicament is a medicament for treating an autoimmune disease with at least one pathologic condition selected from the group consisting of
● hypersensitivity dermatitis (CHS)
● psoriasis
● rheumatoid arthritis;
● morbus bechterew and/or
● inflammatory bowel disease (morbus krohn)

5. Agent according to claim 1 or 2, wherein the CCL17 receptor is at least one receptor selected from the group consisting of
● CCR4,and/or
● CCR8.

6. Agent according to any of the preceding claims, wherein said agent is at least one selected from the group consisting of
● a CCL17 inhibitor,
● an antagonist and/or inhibitor against a CCL17 receptor, preferably against CCR4 and/or CCR8,
● a protein expression inhibitor capable of inhibiting the expression of at least one gene encoding for CCL17 and/or a CCL17 receptor, preferably CCR4 and/or CCR8, and/or
● a degrading agent capable of degrading CCL17 and/or a CCL17 receptor, preferably CCR4 and/or CCR8.

7. Agent according to claim 4, wherein said agent is a monoclonal antibody (mAb).

8. Agent according to claim claim 5, wherein said monoclonal antibody is at least one selected from the group consiting of
● murine, chimeric, humanized and/or human mAb;
● IgG, F(ab)2, Fab, and/or scFv; and/or
● modified antibody format.

9. Agent according to any of the preceding claims, wherein said agent is at least one non-antibody-based target binding entity.

10. Agent according to any of the preceding claims, wherein said agent is a pan receptor antagonist.

11. Agent according to any of the preceding claims, wherein said agent is a small molecular drug.

12. Use of an Agent according to any of the aformentioned claims for the preparation of a medicament.

13. A non-human mammal being partially or completely deficient in expressing the non-human genes encoding CCL17 and apolipoprotein E.

14. The non-human mammal according to claim 11, **characterized in that** said deficiency is due to genetic alteration based on a homozygous or heterozygous mutation in the non-human genes encoding CCL17 and apolipoprotein E; or a homozygous or heterozygous deletion of the non-human genes encoding CCL17 and apolipoprotein E.


SEQUENCE LISTING

<110> Rheinisch Westfälische Technische Hochschule Aachen

<120> CCL17 INHIBITORS FOR USE IN T HELPER CELL-DRIVEN DISEASES

<130> UD40189

<160> 22

<170> PatentIn version 3.3

<210> 1
<211> 20
<212> DNA
<213> Artificial

<220>
<223> Il-2 Forward

<400> 1
cctgagcagg atggagaatt 20

<210> 2
<211> 19
<212> DNA
<213> Artificial

<220>
<223> Il-2 Reverse

<400> 2
tccagaacat gccgcagag 19

<210> 3
<211> 20
<212> DNA
<213> Artificial

<220>
<223> Ifn-gamma Forward

<400> 3
tggctctgca ggattttcat 20

<210> 4
<211> 20
<212> DNA
<213> Artificial

<220>
<223> Ifn-gamma Reverse

<400> 4
tcaagtggca tagatgtgga 20

<210> 5
<211> 22
<212> DNA
<213> Artificial

<220>
<223> Il-4 Forward

<400> 5

caacgaagaa caccacagag ag 22

<210> 6
<211> 22
<212> DNA
<213> Artificial

<220>
<223> Il-4 Reverse

<400> 6
atgaatccag gcatcgaaaa gc 22

<210> 7
<211> 21
<212> DNA
<213> Artificial

<220>
<223> Il-10 Forward

<400> 7
tgcactacca aagccacaag g 21

<210> 8
<211> 22
<212> DNA
<213> Artificial

<220>
<223> Il-10 Reverse

<400> 8
tgggaagtgg gtgcagttat tg 22

<210> 9
<211> 20
<212> DNA
<213> Artificial

<220>
<223> Il-12a Forward

<400> 9
ctgtggtctt cagcaggttt 20

<210> 10
<211> 20
<212> DNA
<213> Artificial

<220>
<223> Il-12a Reverse

<400> 10
cacgctacct cctcttttg 20

<210> 11
<211> 20
<212> DNA
<213> Artificial

<220>

<223>  Il-17a Forward

<400>  11
cctaagaaac ccccacgttt                                                    20


<210>  12
<211>  20
<212>  DNA
<213>  Artificial

<220>
<223>  Il-17a Reverse

<400>  12
cagtttggga cccctttaca                                                    20


<210>  13
<211>  20
<212>  DNA
<213>  Artificial

<220>
<223>  Foxp3 Forward

<400>  13
ttggtttact cgcatgttcg                                                    20


<210>  14
<211>  23
<212>  DNA
<213>  Artificial

<220>
<223>  Foxp3 Reverse

<400>  14
agggattgga gcagcacttg ttg                                                23


<210>  15
<211>  20
<212>  DNA
<213>  Artificial

<220>
<223>  Tgf-beta Forward

<400>  15
tgacgtcact ggagttgtac                                                    20


<210>  16
<211>  20
<212>  DNA
<213>  Artificial

<220>
<223>  Tgf-beta Reverse

<400>  16
ggttcatgtc atggatggtg                                                    20


<210>  17
<211>  20
<212>  DNA

<213>  Artificial

<220>
<223>  CCR4 Forward

<400>  17
ctgacatgtc cagctcctct                                                    20


<210>  18
<211>  20
<212>  DNA
<213>  Artificial

<220>
<223>  CCR4 Reverse

<400>  18
gagcttgtta acgccatgtt                                                    20


<210>  19
<211>  20
<212>  DNA
<213>  Artificial

<220>
<223>  CCR8 Forward

<400>  19
gctgtctatg ccatcaaggt                                                    20


<210>  20
<211>  20
<212>  DNA
<213>  Artificial

<220>
<223>  CCR8 Reverse

<400>  20
cttcagaggc cacttggtaa                                                    20


<210>  21
<211>  20
<212>  DNA
<213>  Artificial

<220>
<223>  Gapdh Forward

<400>  21
ccatcaccat cttccaggag                                                    20


<210>  22
<211>  20
<212>  DNA
<213>  Artificial

<220>
<223>  Gapdh Reverse

<400>  22
gtggttcaca cccatcacaa                                                    20